(19) Europäisches Patentamt
European Patent Office
Office européen des brevets

(11) Publication number: **0 047 546**
**B1**

(12) # EUROPEAN PATENT SPECIFICATION

(45) Date of publication of patent specification: 07.03.84

(51) Int. Cl.³: C 07 D 277/40

(21) Application number: 81200903.3

(22) Date of filing: 13.08.81

(54) **A new method of preparing 3-(2-hydroxy-2-arylethyl)-2-iminothiazolidines.**

(30) Priority: 04.09.80 US 183886

(43) Date of publication of application:
17.03.82 Bulletin 82/11

(45) Publication of the grant of the patent:
07.03.84 Bulletin 84/10

(84) Designated Contracting States:
AT BE CH DE FR GB IT LI LU NL SE

(56) References cited:
DE - C - 664 789
GB - A - 1 109 150
US - A - 3 242 187
US - A - 3 274 209
US - A - 3 804 847
US - A - 3 855 234

Austr. J. Chem. 21 (1968) P. 1557-70

**The file contains technical information submitted after the application was filed and not included in this specification**

(73) Proprietor: JANSSEN PHARMACEUTICA N.V.
Turnhoutsebaan 30
B-2340 Beerse (BE)

(72) Inventor: Albert, Rudolf Willem Hortense
Vekestraat 3
B-2800 Mechelen (BE)
Inventor: De Knaep, Alfons Gaston Maria
Pausenstraat 18
B-2360 Oud-Turnhout (BE)
Inventor: Dockx, Jozef Carolus Joannes
Everweg 14
B-2350 Vosselaar (BE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 047 546 B1

A new method of preparing 3-(2-hydroxy-2-arylethyl)-2-iminothiazolidines

Background of the invention

2,3,5,6-Tetrahydro-6-arylimidazo[2,1-b]thiazoles and the pharmaceutically acceptable acid addition salts thereof are described in U.S. Patent No. 3,274,209. The compounds are powerful anthelminthic agents. The best known member of this imidazo[2,1-b]thiazole group is 2,3,5,6-tetrahydro-6-phenylimidazo[2,1-b]thiazole and the laevo isomer thereof, levamisole, which is described in U.S. Patent No. 3,463,786.

The preparation of tetramisole by cyclizing 3-(2-hydroxy-2-phenylethyl)-2-iminothiazolidine of formula

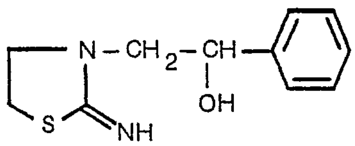

(i)

as well as the preparation of said thiazolidine (i), starting from 2-phenyloxirane and 2-aminoethanol, is described in U.S. Patent No. 3,855,234. The preparation of the compound of formula (i), starting from 2-phenyloxirane and 2-iminothiazolidine is described in British Patent No. 1,109,150.

In comparison with the prior art methods, the new method for the manufacture of 3-(2-hydroxy-2-arylethyl)-2-iminothiazolidine, according to the present invention, has the advantage of being less polluting, because of the absence of thionyl chloride in the reaction scheme.

Moreover, due to the availability and the low cost prices of the starting materials and the absence of undesired reactions, the novel method has, in comparison with the prior art methods, a more favorable overall economy.

Description of the preferred embodiments:

This invention is concerned with a novel process for the manufacture of a chemical compound having the formula

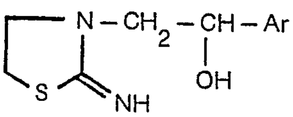

(I)

and acid addition salts thereof,

wherein Ar is a member selected from the group consisting of phenyl, halophenyl, nitrophenyl and (trifluoromethyl)phenyl, by protonating a starting material having the formula

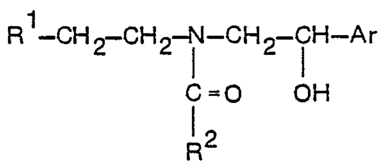

(II)

wherein Ar is as above described, $R^1$ is chloro or bromo and $R^2$ is a lower alkyloxy, aryloxy or an optionally substituted phenyl radical, or, $R^1$ and $R^2$, when taken together, represent an —O— radical. and subsequently reacting the thus obtained protonated intermediate with a reagent selected from the group consisting of thiourea, ammonium isothiocyanate and the alkali- and earth alkaline metal isothiocyanates.

In the foregoing definitions the term "halo" is generic to fluoro, chloro, bromo and iodo, and "lower alkyl" is meant to include straight and branched saturated hydrocarbon radicals having from 1 to 6 carbon atoms such as, for example, methyl, ethyl, 1-methylethyl, 1,1-dimethylethyl, propyl, 2-methylpropyl, butyl, pentyl, hexyl and the like.

Although the protonation reaction is preferably carried out in anhydrous medium, suitable organic solvents may also be used in admixture with water, thus creating a homogeneous or a heterogeneous mixture. Suitable organic solvents are, for example, aliphatic-, alicyclic- and aromatic hydrocarbons, e.g., pentane, cyclohexane, methylbenzene and the like, alcohols, e.g., methanol, 2-propanol and the like, ethers, e.g., tetrahydrofuran, 1,4-dioxane and the like, halogenated hydrocarbons, e.g., trichloromethane and the like, or a mixture of said solvents.

Suitable protonating agents are strong non-oxidizing acids such as, for example, hydrobromic acid, hydrochloric acid, 4-methylbenzenesulfonic acid and the like. When the protonation is carried out in a non-aqueous medium, anhydrous acids are most preferred. Suitable anhydrous acids are, for example, gazeous hydrochloric acid and anhydrous 4-methylbenzenesulfonic acid.

Preferably, the protonation reaction is carried out at temperatures comprised between 60°C and 130°C and, most preferably, the reaction is carried out at the reflux temperature of the reaction mixture.

The conversion of the protonated intermediate with thiourea, ammonium isothiocyanate or an alkali- or earth alkaline metal isothiocyanate may be carried out by stirring the reactants together in a suitable acidic reaction medium. The reaction may be accelerated or completed when said conversion is carried out at temperatures comprised between 40°C and 100°C. Preferably the reaction is carried out in an aqueous medium. Most preferably, the reaction is carried out at the reflux temperature of the reaction mixture.

In a particularly preferred method, the protonation reaction and the reaction with thiourea, ammonium isothiocyanate or an alkali- or earth alkaline metal isothiocyanate are carried out by firstly bubbling dry gazeous hydrochloric acid through a solution of an appropriate starting material of formula (II) in a suitable anhydrous organic solvent, at temperatures comprised between 70°C and 120°C, subsequently adding water and a sufficient amount of thiourea or an appropriate isothiocyanate and finally refluxing the reaction mixture for from 1 to 48 hours.

The 3-(2-hydroxy-2-phenylethyl)-2-iminothiazolidine (I) may be converted into acid addition salt forms by treatment with an appropriate acid such as, for example, an inorganic acid, such as, hydrohalic acid, e.g., hydrochloric, hydrobromic and the like, and sulfuric acid, nitric acid, phosphoric acid and the like; or an organic acid such as, for example, acetic, propanoic, 2-hydroxyacetic, 2-hydroxypropanoic, 2-oxopropanoic, propanedioic, butanedioic, (Z)-2-butenedioic, (E)-2-butenedioic, 2-hydroxybutanedioic, 2,3-dihydroxybutanedioic, 2-hydroxy-1,2,3-propanetricarboxylic, benzoic, 3-phenyl-2-propenoic, α-hydroxybenzeneacetic, methanesulfonic, ethanesulfonic, benzenesulfonic, 4-methylbenzenesulfonic, cyclohexanesulfamic, 2-hydroxybenzoic, 4-amino-2-hydroxybenzoic and the like acids.

Conversely the salt form can be converted by treatment with alkali into the free base form.

The compounds of formula (II) are novel, and, as useful starting materials in the process of the present invention, they constitute an additional feature thereof

Said compounds of formula (II) may be derived from the corresponding halides of formula

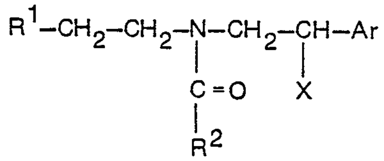

(III)

wherein $R^1$, $R^2$ and Ar are as previously described and X is generic to chloro, bromo and iodo, by stirring the halide (III) in water, if desired, in admixture with a water-miscible solvent such as, for example, an alcohol, e.g., methanol, 2-propanol and the like, a cyclic ether, e.g., tetrahydrofuran, 1,4-dioxane and the like.

Elevated temperatures may be used to enhance the rate of the reaction and, most preferably, the reaction is carried out at the reflux temperature of the reaction mixture.

The compounds of formula (III), used as starting materials in the above described reaction, constitute as novel compounds and as useful intermediates herein an additional feature of this invention.

The starting materials of formula (III) may be prepared by reacting an appropriately substituted ethenylarene, having the formula

$$Ar—CH{=}CH_2 \quad (IV),$$

wherein Ar is as previously described, with a compound of formula

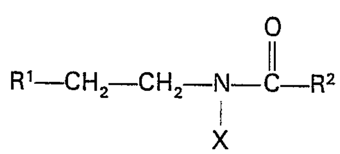

(V),

wherein $R^1$, $R^2$ and X are as previously described, in the presence of an appropriate radical initiator in a suitable reaction-inert solvent.

Suitable solvents are, for example, aliphatic-, alicyclic- and aromatic hydrocarbons, e.g., hexane, cyclohexane, benzene and the like, halogenated hydrocarbons, e.g., di-, tri-, tetrachloromethane and the like.

Appropriate radical initiators are anorganic or organic compounds, which decompose at moderate temperatures and/or under irradiation, such as, for example, azo compounds, e.g., 2,2′-azabis[2-methylpropanenitrile] and the like.

Elevated temperatures may be used to enhance the rate of the reaction and, preferably, the reaction is carried out at a temperature comprised between room temperature and 100°C.

The starting compounds of formula (V) are generally known. For example, the compounds of formula (V), wherein $R^1$ and $R^2$, taken together, form an —O— radical, are described in U.S. Patent No. 3,591,601.

The compounds of formula (II) may also be prepared by reacting an appropriately substituted 2-aryloxyirane of formula

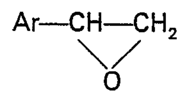

(VI)

with a compound having the formula

$$R^1—CH_2—CH_2—NH—CO—R^2 \qquad (VII)$$

wherein $R^1$ and $R^2$ are as previously described,
in the presence of an appropriate base such as, for example, alkali or earth alkaline metal hydrides or hydroxides, e.g., sodium hydroxide, sodium hydride and the like, in a suitable reaction-inert solvent.

Suitable solvents, depending on the nature of the base are, e.g., water, benzene, N,N-dimethylformamide and the like.

The starting compounds (VI) and (VII) are generally known in the art.

The hereinabove described reactions have the advantage that they can all be carried out without isolating any intermediately formed compounds.

The nomenclature, used in the foregoing specification, the examples and the claims are in correspondence with the rules described in Chemical Abstracts, Volume *76*, Index Guide, pages 21 I—140 I.

The following examples are intended to illustrate and not to limit the scope of the present invention. Unless otherwise stated all parts therein are by weight.

### Example I

Preparation of 3-bromo-2-oxazolidinone

To a solution of 43.6 parts of 2-oxazolidinone in 500 parts of water there are added, over a period of 25 minutes, 80 parts of bromine, while the pH of the reaction mixture is directed between pH 8 and 10.5 by adding 10.4 N aqueous sodium hydroxide. Upon completion, the reaction mixture is stirred during 5 minutes. The residue is filtered off, washed several times with water and dried, yielding 56.2 parts (66.0%) of 3-bromo-2-oxazolidinone. the filtrate is extracted several times with dichloromethane. The combined organic fractions are dried, filtered and evaporated, yielding 11.6 parts (14.7%) of 3-bromo-2-oxazolidinone, thus obtaining an overall yield of 80.7%.

### Example II

Preparation of 3-(2-bromo-2-arylethyl)-2-oxazolidinone

While nitrogen gas is introduced, 17.5 parts of 3-bromo-2-oxazolidinone are added to a warmed suspension (60°C) of 0.82 parts of 2,2′-azabis[2-methylpropanenitrile] in 105 parts of benzene. During a period of 5 minutes, a solution of 10.4 parts of ethenylbenzene in 17 parts of benzene is added. Upon completion, the reaction mixture is stirred at room temperature overnight and the solution is evaporated. The residue is taken up in 35 parts of petroleumether, filtered and dried, yielding 27.22 parts (96.8%) of 3-(2-bromo-2-phenylethyl)-2-oxazolidinone; mp. 88.6°C.

Following the same procedure and using equivalent amounts of the appropriate starting materials, there is also prepared: 3-[2-bromo-2-(3-nitrophenyl)ethyl]-2-oxazolidinone; mp. 96.4°C.

### Example III

Preparation of 3-(2-hydroxy-2-arylethyl)-2-oxazolidinone

A mixture of 8.67 parts of 3-(2-bromo-2-phenylethyl)-2-oxazolidinone in 30 parts of water is stirred and refluxed during 30 minutes. While cooling in an ice-bath, an oil, which solidifies on standing, precipitates. The solid is filtered off, washed with water and dried with calcium chloride at 50°C, yielding 5.61 parts of 3-(2-hydroxy-2-phenylethyl)-2-oxazolidinone. Crystallization of 5 parts of 3-(2-hydroxy-2-phenylethyl)-2-oxazolidinone from 16 parts of 2-propanol (activated charcoal) yields 4.2 parts of 3-(2-hydroxy-2-phenylethyl)-2-oxazolidinone. Purification of 4.2 parts of 3-(2-hydroxy-2-phenylethyl)-2-oxazolidinone by column-chromatography, using benzene/ethanol (9:1) as eluent, yields 3.28 parts; mp. 126°C.

Following the same procedure and using equivalent amounts of the appropriate starting materials there is also prepared:
3-[2-hydroxy-2-(3-nitrophenyl)ethyl]-2-oxazolidinone; mp. 158.9°C.

Example IV

a) Preparation of ethyl [(2-chloroethyl)(2-bromo-2-phenylethyl)]carbamate.

Over a period of 55 minutes, 16 parts of bromine are added dropwise to a stirred mixture of 16 parts of ethyl (2-chloroethyl)carbamate and 100 parts of water, while the reaction mixture is maintained at pH 9 by adding a sodium hydroxide solution 1N. The reaction mixture is extracted with dichloromethane. The organic phase is dried, filtered and evaporated at room temperature. The residue is dissolved in 80 parts of benzene and used as such in the following reaction.

To the hereinabove described solution is added 0.63 parts of 2,2′-azabis[2-methylpropanenitrile] while stirring under nitrogen atmosphere, and the whole is warmed up to 40°C. A solution of 8 parts of freshly distilled ethenylbenzene in 12 parts of benzene is added over a period of 8 minutes and stirring is continued at 60°C for 6 hours. The whole is evaporated, yielding quantitatively ethyl [(2-chloroethyl)(2-bromo-2-phenylethyl)]carbamate.

b) Preparation of ethyl [(2-chloroethyl)(2-hydroxy-2-phenylethyl)]carbamate.

A mixture of 29.0 parts of ethyl [(2-chloroethyl)(2-bromo-2-phenylethyl)]carbamate in 100 parts of water is stirred and refluxed during 30 minutes. After cooling at room temperature, the aqueous solution is extracted several times with trichloromethane. The combined organic layers are washed with water, dried and evaporated, yielding 26.1 parts of ethyl [(2-chloroethyl)(2-hydroxy-2-phenylethyl)]carbamate as an oily residue.

Example V

Preparation of 3-(2-hydroxy-2-arylethyl)-2-iminothiazolidine

i) Dry gaseous hydrochloric acid is bubbled during 28 hours through a warmed (100°C) solution of 4.15 parts of 3-(2-hydroxy-2-phenylethyl)-2-oxazolidinone in 35 parts of methylbenzene. After cooling (60°C), 20 parts of water and 2.3 parts of thiourea are added and the mixture is refluxed during 16 hours. 3.8 Parts of 4-methylbenzenesulfonic acid are added. The mixture is cooled at room temperature and filtered. The residue is washed with 12 parts of 2-propanone and dried, yielding 4.53 parts (57.4%) of 3-(2-hydroxy-2-phenylethyl)-2-iminothiazolidine 4-methylbenzenesulfonate (1:1); mp. 232—235°C.

Following the same procedure and using equivalent amounts of the appropriate starting materials, there is also prepared:

3-[2-hydroxy-2-(3-nitrophenyl)ethyl]-2-iminothiazolidine 4-methylbenzenesulfonate (1:1); mp. 237.1°C.

ii) Dry gazeous hydrochloric acid is bubbled during 28 hours through a warmed (100°C) solution of 15.3 parts of ethyl [(2-chloroethyl)(2-hydroxy-2-phenylethyl)]carbamate in 120 parts of methylbenzene. After cooling (60°C), 60 parts of water and 6.1 parts of thiourea are added and the mixture is refluxed during 16 hours. 9.5 Parts of 4-methylbenzenesulfonic acid are added, the mixture is cooled at room temperature and filtered. The residue is washed with 20 parts of 2-propanone and dried, yielding 6.7 parts (60%) of 3-(2-hydroxy-2-phenylethyl)-2-iminothioazolidine 4-methylbenzenesulfonate (1:1); mp. 232—235°C.

**Claims**

1. A process for the manufacture of a chemical compound having the formula

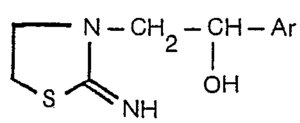

(I)

and acid addition salts thereof,

wherein Ar is a member selected from the group consisting of phenyl, halophenyl, nitrophenyl and (trifluoromethyl)phenyl, characterized by protonating a starting material having the formula

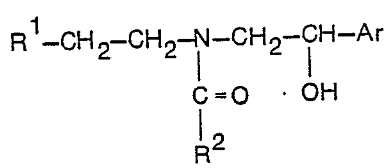

(II),

wherein $R^1$ is chloro or bromo and $R^2$ is a $C_1$—$C_6$ alkyloxy radical, an aryloxy radical or an optionally substituted phenyl radical, or, $R^1$ and $R^2$, when taken together, represent an —O— radical, and subsequently reacting the thus obtained protonated intermediate with a reagent selected from the group consisting of thiourea, ammonium isothiocyanate and the alkali- and earth alkaline metal isothiocyanates.

2. A process according to claim 1 wherein the protonating agent is dry gazeous hydrochloric acid.

3. A process according to claim 1 wherein the reaction with thiourea, ammonium isothiocyanate or an alkali- or earth alkaline metal isothiocyanate is carried out in a suitable acidic reaction medium.

4. A process according to claim 1 characterized in that it comprises the steps of

a) bubbling dry gazeous hydrochloric acid through a solution of an appropriate starting material having the formula

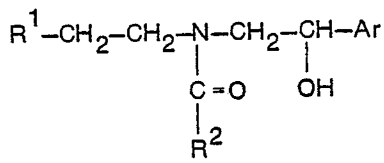

(II)

in a suitable anhydrous organic solvent at a temperature comprised between 70°C and 120°C; and

b) subsequently adding water and a sufficient amount of thiourea, ammonium isothiocyanate, or an alkali- or earth alkaline metal isothiocyanate and refluxing the reaction mixture for from 1 to 48 hours.

5. A process according to claim 4 further characterized in that the starting material of the formula (II) is derived from a halide, having the formula

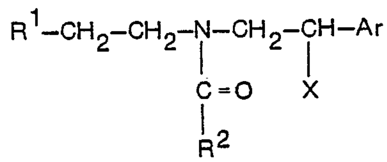

(III),

wherein X is generic to chloro, bromo and iodo,
by stirring the halide of formula (III) in water, if desired, in admixture with a water-miscible solvent.

6. A process according to claim 4 characterized in that the starting material of formula (II) therein is prepared by

a) reacting an appropriate ethenylarene of formula

$$Ar—CH=CH_2 \qquad (IV)$$

with a compound of formula

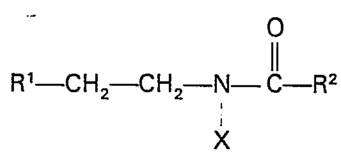

(V)

wherein X is generic to chloro, bromo and iodo, $R^1$ is chloro or bromo and $R^2$ is a $C_1$—$C_6$ alkyloxy radical, an aryloxy radical or an optionally substituted phenyl radical, or, $R^1$ and $R^2$, when taken together, represent an —O— radical, in a suitable reaction-inert solvent in the presence of an appropriate radical initiator at a temperature comprised between room temperature and 100°C; and

b) hydrolyzing the thus obtained halide of formula

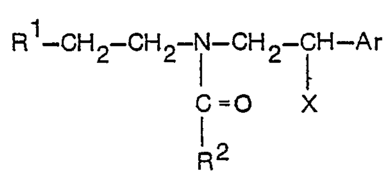

(III)

by stirring said halide (III) in acidic aqueous medium at the reflux temperature of the reaction mixture.

7. A process according to claim 4 further characterized in that the starting alcohol of formula (II) is prepared by reacting an appropriately substituted 2-aryloxirane of formula

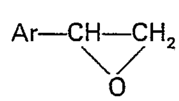

(VI)

with a compound having the formula

$$R^1—CH_2—CH_2—NH—CO—R^2 \qquad (VII)$$

in the presence of an appropriate base in a suitable reaction-inert solvent.

8. A process according to claim 4 wherein the starting material is a 2-oxazolidinone, having the formula

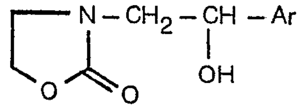

(II')

9. A process according to claim 4 wherein the starting material is 3-(2-hydroxy-2-phenylethyl)-2-oxazolidinone.

10. A chemical compound selected from the group consisting of an alcohol having the formula

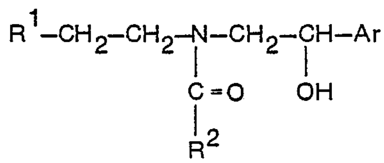

(II),

wherein $R^1$ is chloro or bromo; $R^2$ is a $C_1$—$C_6$ alkyloxy radical, an aryloxy radical or an optionally substituted phenyl radical, or $R^1$ and $R^2$, when taken together, represent an —O— radical; and Ar is a member selected from the group consisting of phenyl halophenyl, nitrophenyl and (trifluoromethyl)phenyl.

11. A chemical compound according to claim 10 wherein $R^1$ and $R^2$ together form an —O— radical.

12. A chemical compound: 3-(2-hydroxy-2-phenylethyl)-2-oxazolidinone.

13. A chemical compound selected from the group consisting of halides having the formula

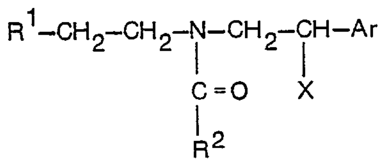

(III)

wherein $R^1$ is chloro or bromo; $R^2$ is a $C_1$—$C_6$ alkyloxy radical, an aryloxy radical or an optionally substituted phenyl radical, or, $R^1$ and $R^2$, when taken together, represent an —O— radical;

Ar is a member selected from the group consisting of phenyl, halophenyl, nitrophenyl and (trifluoromethyl)phenyl; and X is chloro, bromo or iodo.

14. A chemical compound according to claim 13 wherein $R^1$ and $R^2$ together form an —O— radical.

15. A method of preparing tetramisole or levamisole starting from an intermediate of the formula

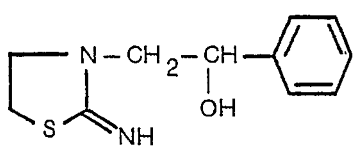

(i)

characterized in that said intermediate of formula (i) has been prepared by a process as claimed in claim 1.

## Patentansprüche

1. Ein Verfahren zur Herstellung einer chemischen Verbindung mit der Formel

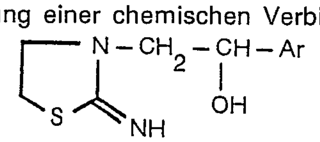

(I)

und deren Säureadditionssalzen, worin Ar ein Glied, ausgewählt aus der aus Phenyl, Halogenphenyl, Nitrophenyl und (Trifluormethyl)phenyl bestehenden Gruppe, bedeutet, dadurch gekennzeichnet, daß ein Ausgangsmaterial mit der Formel

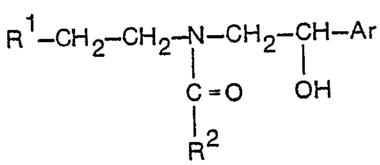

(II)

worin $R^1$ Chlor oder Brom bedeutet und $R^2$ für einen $C_1$—$C_6$-Alkyloxy-Rest. einen Aryloxy-Rest oder einen gegebenenfalls substituierten Phenylrest steht oder $R^1$ und $R^2$ gemeinsam einen —O—Rest bedeuten, protonisiert wird und anschließend das solcherart erhaltene protonisierte Zwischenprodukt mit einem Reaktionsmittel, ausgewählt aus der aus Thioharnstoff, Ammonium-isothiocyanat und den Alkali- und Erdalkalimetall-isothiocyanaten bestehenden Gruppe, umgesetzt wird.

2. Ein Verfahren nach Anspruch 1, worin das protonisierende Mittel trockene, gasförmige Chlorwasserstoffsäure ist.

3. Ein Verfahren nach Anspruch 1, worin das Umsetzung mit Thioharnstoff, Ammonium-isothiocyanat oder Alkali- oder Erdalkalimetall-isothiocyanat in einem geeigneten sauren Reaktionsmedium ausgeführt wird.

4. Ein Verfahren nach Anspruch 1, dadurch gekennzeichnet, daß es die Stufen des

a) Durchleitens von trockener gasförmiger Chlorwasserstoffsäure durch eine Lösung eines entsprechenden Ausgangsmaterials der Formel

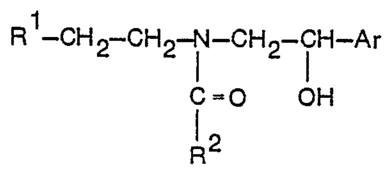

, (II)

in einem geeigneten wasserfreien organischen Lösungsmittel bei einer Temperatur zwischen 70°C und 120°C und

b) eines anschließnden Zusetzens von Wasser und einer ausreichenden Menge von Thioharnstoff, Ammonium-isothiocyanat oder einem Alkali- oder Erdalkalimetall-isothiocyanat und des Rückflußsiedens des Reaktionsgemisches während 1 bis 48 Stunden umfaßt. Stunden umfaßt.

5. Ein Verfahren nach Anspruch 4, weiterhin dadurch gekennzeichnet, daß das Ausgangsmaterial der Formel (II) von einem Halogenid mit der Formel

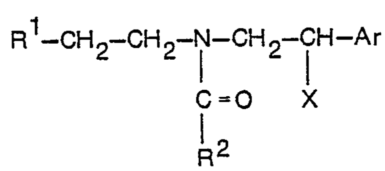

, (III)

worin X für Chlor, Brom oder Jod steht, durch Rühren des Halogenids der Formel (III) in Wasser, gewünschtenfalls im Gemisch mit einem wassermischbaren Lösungsmittel, abgeleitet wird.

6. Ein Verfahren nach Anspruch 4, dadurch gekennzeichnet, daß das darin erwähnte Ausgangsmaterial der Formel (II) hergestellt wird durch

a) Umsetzen eines entsprechenden Äthenylarens der Formel

$$Ar—CH=CH_2 \qquad (IV)$$

mit einer Verbindung der Formel

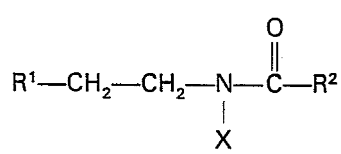

(V)

worin X für Chlor, Brom oder Jod steht. $R^1$ Chlor oder Brom bedeutet und $R^2$ einen $C_1$—$C_6$-Alkyloxy-Rest, einen Aryloxy-Rest oder einen gegebenenfalls substituierten Phenylrest bedeutet oder $R^1$ und $R^2$ zusammen einen —O—Rest bedeuten, in einem geeigneten reaktionsinerten Lösungsmittel in Gegenwart eines entsprechenden Radikal-Initiators bei einer Temperatur zwischen Raumtemperatur und 100°C; und

b) Hydrolysieren des solcherart erhaltenen Halogenids der Formel

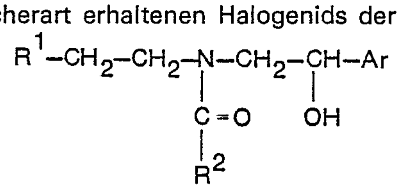

(III)

durch Rühren des genannten Halogenids (III) in einem sauren wässerigen Medium bei der Rückflußtemperatur des Reaktionsgemisches.

7. Ein Verfahren nach Anspruch 4, weiterhin dadurch gekennzeichnet, daß der Ausgangsalkohol der Formel (II) hergestellt wird durch Umsetzen eines entsprechend substituierten 2-Aryloxirans der Formel

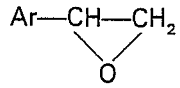

(VI)

mit einer Verbindung der Formel

$$R^1—CH_2—CH_2—NH—CO—R^2$$

(VII)

in Gegenwart einer entsprechenden Base in einem geeigneten reaktionsinerten Lösungsmittel.

8. Ein Verfahren nach Anspruch 4, worin das Ausgangsmaterial ein 2-Oxazolidinon mit der Formel

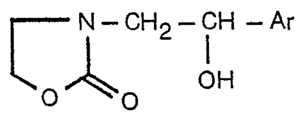

(II')

ist.

9. Ein Verfahren nach Anspruch 4, worin das Ausgangsmaterial 3-(2-Hydroxy-2-phenyläthyl)-2-oxazolidinon ist.

10. Eine chemische Verbindung, ausgewählt aus der aus einem Alkohol mit der Formel

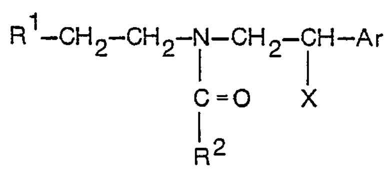

(II)

bestehenden Gruppe, worin $R^1$ Chlor oder Brom ist; $R^2$ einen $C_1$—$C_6$-Alkyloxy-Rest, einen Aryloxy-Rest oder einen gegebenenfalls substituierten Phenylrest bedeutet oder $R^1$ und $R^2$ zusammen einen —O—Rest bedeuten; und Ar ein Glied aus der Gruppe, bestehend aus Phenyl, Halogenphenyl, Nitrophenyl und (Trifluormethyl)phenyl, darstellt.

11. Eine chemische Verbindung nach Anspruch 10, worin $R^1$ und $R^2$ zusammen einen —O—Rest bilden.

12. Eine chemische Verbindung: 3-(2-Hydroxy-2-phenyläthyl)-2-oxazolidinon.

13. Eine chemische Verbindung, ausgewählt aus der Gruppe, bestehend aus Halogeniden der Formel

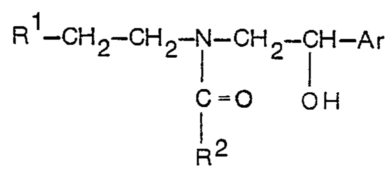

(III)

worin $R^1$ Chlor oder Brom bedeutet; $R^2$ einen $C_1$—$C_6$-Alkyloxy-Rest; einen Aryloxy-Rest oder einen gegebenenfalls substituierten Phenylrest bedeutet oder $R^1$ und $R^2$ zusammen einen —O—Rest bedeuten; Ar ein Glied, ausgewählt aus der aus Phenyl, Halogenphenyl, Nitrophenyl und (Trifluormethyl)phenyl bestehenden Gruppe, darstellt; und X für Chlor, Brom oder Jod steht.

14. Eine chemische Verbindung nach Anspruch 13, worin $R^1$ und $R^2$ gemeinsam einen —O—Rest bilden.

15. Ein Verfahren zur Herstellung von Tetramisol oder Levamisol, ausgehend von einem Zwischenprodukt der Formel

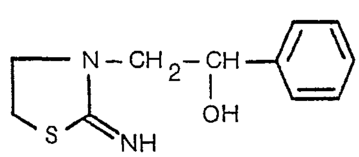

(i)

dadurch gekennzeichnet, daß das genannte Zwischenprodukt der Formel (i) nach einem Verfahren hergestellt worden ist, wie es in Anspruch 1 beansprucht ist.

**Revendications**

1. Un procédé pour la fabrication d'un composé chimique ayant pour formule

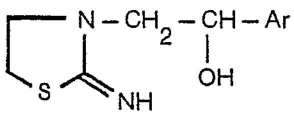

(I)

et de ses sels d'addition d'acides,

où Ar est choisi parmi le groupe constitué par un phényle, un halogénophényle, un nitrophényle et un (trifluorométhyl)phényle, caractérisé par la protonation d'une matière de départ ayant pour formule

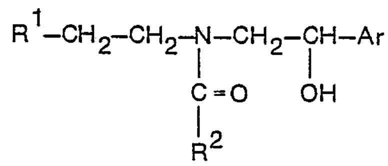

(II)

où $R^1$ est un chloro ou un bromo et $R^2$ est un radical alcoxy en $C_1$—$C_6$, un radical aryloxy ou un radical phényle éventuellement substitué, ou $R^1$ et $R^2$, pris ensemble, représentant un radical —O—, puis la réaction de l'intermédiaire protoné ainsi obtenu avec un composé réagissant choisi parmi le groupe constitué par la thio-urée, l'isothiocyanate d'ammonium et les isothiocyanates de métaux alcalins et alcalino-terreux.

2. Un procédé selon la revendication 1, dans lequel l'agent de protonation est l'acide chlorhydrique gazeux anhydre.

3. Un procédé selon la revendication 1, dans lequel la réaction avec la thio-urée, l'isothiocyanate d'ammonium ou un isothiocyanate de métal alcalin ou alcalino-terreux est effectuée dans un milieu réactionnel acide approprié.

4. Un procédé selon la revendication 1, caractérisé en ce qu'il comprend les stades de

a) barbotage d'acide chlorhydrique gazeux anhydre à travers une solution d'une matière de départ appropriée ayant pour formule

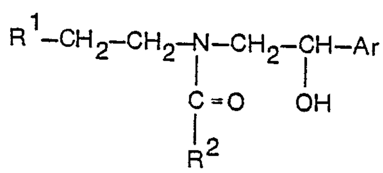

(II)

dans un solvant organique anhydre approprié à une température comprise entre 70°C et 120°C; et

b) l'addition ultérieure d'eau et d'une quantité suffisante de thio-urée, d'isothiocyanate d'ammonium ou d'un isothiocyanate de métal alcalin ou alcalino-terreux et le reflux du mélange réactionnel pendant 1 à 48 heures.

5. Un procédé selon la revendication 4, caractérisé de plus en ce que la matière de départ de formule (II) dérive d'un halogénure ayant pour formule

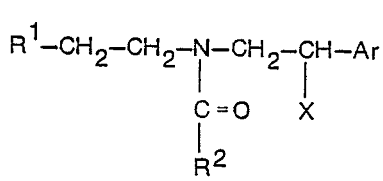

(III)

où X représente de façon générale chloro, bromo ou iodo, par agitation de l'halogénure de formule (III) dans l'eau, si on le désire en mélange avec un solvant miscible à l'eau.

6. Un procédé selon la revendication 4, caractérisé en ce que la matière de départ de formule (II) est préparée par

a) réaction d'un éthénylarène approprié de formule

$$Ar—CH=CH_2 \qquad (IV)$$

avec un composé de formule

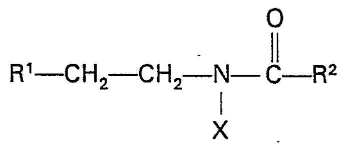

(V)

où X est de façon générale chloro, bromo ou iodo, $R^1$ est chloro ou bromo et $R^2$ est un radical alcoxy en $C_1$—$C_6$, un radical aryloxy ou un radical phényle éventuellement substitué en $R^1$ ou $R^2$,

pris ensemble, représentent un radical —O—, dans un solvant approprié inerte dans la réaction en présence d'un amorceur radicalaire approprié à une température comprise entre la température ordinaire et 100°C; et

b) l'hydrolyse de l'halogénure ainsi obtenu de formule

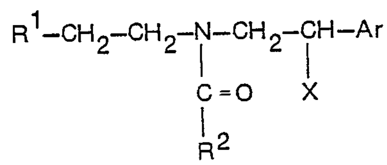

(III)

par agitation dudit halogénure (III) dans un milieu aqueux acide à la température de reflux du mélange réactionnel.

7. Un procédé selon la revendication 4, caractérisé de plus en ce que l'alcool de départ de formule (II) est préparé par réaction d'un 2-aryloxiranne convenablement substitué de formule

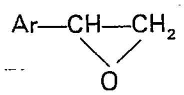

(VI)

avec un composé ayant pour formule

$$R^1—CH_2—CH_2—NH—CO—R^2 \qquad (VII)$$

en présence d'un base appropriée dans un solvant approprié inerte dans la réaction.

8. Un procédé selon la revendication 4, dans lequel la matière de départ est une 2-oxazolidinone ayant pour formule

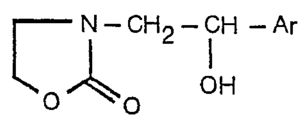

(III)

9. Un procédé selon la revendication 4, dans lequel la matière de départ est la 3-(2-hydroxy-2-phényléthyl)-2-oxazolidinone.

10. Un composé chimique choisi parmi le groupe constitué par un alcool ayant pour formule

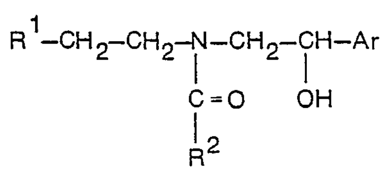

(II)

où $R^1$ est un chloro ou un bromo; $R^2$ est un radical alcoxy en $C_1$—$C_6$, un radical aryloxy ou un radical phényle éventuellement substitué ou, $R^1$ et $R^2$, pris ensemble, représentent un radical —O—; et Ar est choisi parmi le groupe constitué par un phényle, un halogénophényle, un nitrophényle et un (trifluorométhyl)phényle.

11. Un composé chimique selon la revendication 10, dans lequel $R^1$ et $R^2$ forment ensemble un radical —O—.

12. Un composé chimique: la 3-(2-hydroxy-2-phényléthyl)-2-oxazolidinone.

13. Un composé chimique choisi parmi le groupe constitué par les halogénures ayant pour formule

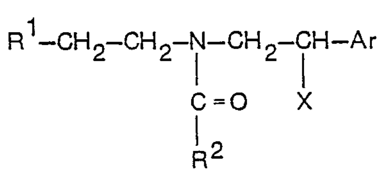

(III)

où $R^1$ est un chloro ou un bromo; $R^2$ est un radical alcoxy en $C_1$—$C_6$, un radical aryloxy ou un radical

phényle éventuellement substitué ou, $R^1$ et $R^2$, pris ensemble, représentent un radical —O—; Ar est choisi parmi le groupe constitué par un phényle, un halogénophényle, un nitrophényle et un (trifluorométhyl)phényle; et X est un chloro, bromo ou iodo.

14. Un composé chimique selon la revendication 13, dans lequel $R^1$ et $R^2$ forment ensemble un radical —O—.

15. Un procédé de préparation de tétramisole ou de lévamisole à partir d'un intermédiaire de formule

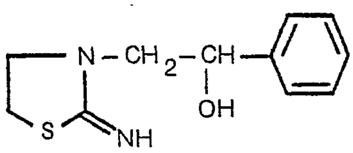

(i)

caractérisé en ce que ledit intermédiaire de formule (i) a été préparé selon un procédé comme revendiqué dans la revendication 1.